# FASCICULE DE BREVET EUROPEEN

(11) **EP 3 687 455 B1**
(45) Date de publication et mention de la délivrance du brevet: **24.11.2021**
(21) Numéro de dépôt: 18773488.4
(22) Date de dépôt: 28.09.2018
(51) Int. Cl.: A61F 2/42, A61B 17/17, A61B 17/86, A61F 2/30

(54) **PROTHÈSE DE CHEVILLE COMPORTANT UN IMPLANT TALIEN, UN IMPLANT TIBIAL ET UN INSERT, ET KIT INCLUANT AU MOINS UNE TELLE PROTHÈSE**
KNÖCHELPROTHESE MIT EINEM TALUSIMPLANTAT, EINEM TIBIAIMPLANTAT UND EINEM EINSATZ SOWIE KIT MIT MINDESTENS EINER SOLCHEN PROTHESE
ANKLE PROSTHESIS COMPRISING A TALAR IMPLANT, A TIBIAL IMPLANT AND AN INSERT, AND KIT INCLUDING AT LEAST ONE SUCH PROSTHESIS

(30) Priorité: 28.09.2017 FR 1758993
(43) Date de publication de la demande: 05.08.2020
(73) Titulaire: Biotechni, 13600 La Ciotat (FR)
(72) Inventeur: CALAMEL, Lionel, 13380 Plan de Cuques (FR); OLIVERA, Franck, 83910 Pourrieres (FR); ASENCIO, Joseph-Guy, 30900 Nimes (FR); DI SCHINO, Marion, 30900 Nimes (FR); LEONARDI, Christian, 30900 Nimes (FR); CALAMEL, Lionel, 13600 La Ciotat (FR)
(74) Mandataire: Lavoix
(86) Numéro de dépôt international: PCT/EP2018/076500
(87) Numéro de publication internationale: WO 2019/063807

(56) Documents cités:
- EP-A1- 2 363 087
- WO-A1-00/69373
- FR-A1- 2 759 900
- US-A1- 2011 035 019

## Description

La présente invention concerne une prothèse de cheville du type comportant un implant talien, un implant tibial et un insert sur lequel s'articulent ces deux implants. L'invention est définie dans les revendications.

On connaît des prothèses de cheville comportant les trois éléments précités. L'une est décrite dans le document WO-A-00/69373.

Elle comporte un implant talien de surface inférieure courbe et portant sur sa face inférieure deux picots orientés obliquement vers l'arrière de la prothèse. Cette surface inférieure constitue l'interface entre l'implant talien et l'os du talon. Les picots servent à l'ancrage de l'implant talien sur l'os du talon. La surface supérieure de l'implant talien est en forme de « selle », c'est-à-dire qu'elle est pourvue d'un sillon longitudinal (autrement dit, s'étendant entre l'avant et l'arrière du corps du patient et de la prothèse, et non entre la droite et la gauche du corps du patient et de la prothèse, comme le ferait un sillon transversal). La section transversale du sillon présente un rayon de courbure et le sillon sépare la surface supérieure de l'implant talien en deux portions connectées l'une à l'autre par des lignes d'inflexion selon lesquelles la direction de courbure s'inverse. Chacune de ces portions a une forme en portion de sphère.

Elle comporte aussi un implant tibial, destiné à être fixé à l'extrémité inférieure du tibia préalablement adéquatement conformée par le chirurgien. Sa surface supérieure est de forme générale sensiblement plane et comporte des moyens d'ancrage osseux constitués par deux cylindres qui s'insèrent dans des logements ménagés dans le tibia à cet effet. Sa surface inférieure est sensiblement sphérique.

Elle comporte, enfin, un insert placé entre les deux implants précédents, et dont les faces inférieure et supérieure ont des formes correspondant à celles des implants respectifs avec lesquels elles sont en contact. Ainsi, d'une part la liaison entre l'insert et l'implant tibial fonctionne à la manière d'une rotule, et d'autre part il y a une possibilité de glissement longitudinal de l'insert sur la surface supérieure de l'implant talien. De cette façon, on reproduit au mieux les mouvements naturels relatifs entre le tibia et l'os talien grâce à la prothèse.

Un autre exemple d'une prothèse de cheville en trois telles parties figure dans le document EP-A-1 915 975. L'implant talien a une surface inférieure courbe et qui comporte deux picots d'ancrage. Cet ancrage sur l'os talien est complété par une patte de fixation antérieure laissant passer des vis et par des flasques latéraux qui encadrent l'os talien. L'implant tibial a sa fixation sur le tibia assurée par des pointes disposées sur la surface supérieure plane de l'implant tibial et par une butée transversale verticale, c'est-à-dire sensiblement perpendiculaire à la surface supérieure plane de l'implant tibial, qui est disposée sur le bord antérieur de l'implant tibial. Elle forme un bouclier tibial qui, en plus de sa fonction de maintien en place de l'implant tibial, empêche un développement inconsidéré de cellules osseuses susceptibles de gêner le fonctionnement de l'articulation. L'insert a une face inférieure dont la géométrie correspond à celle de la face supérieure de l'implant talien et une face supérieure dont la géométrie correspond à celle de la surface inférieure de l'implant tibial. Une particularité de cette prothèse est qu'elle comporte un exemple de moyen d'accouplement configurable conçu pour autoriser le réglage de la position de l'insert par rapport à l'implant tibial. Ce réglage est obtenu entre autre par des moyens d'accouplement tels que des vis de compression qui traversent le bouclier de l'implant tibial et sont dirigées vers l'insert.

Cette dernière configuration de l'implant tibial, avec son bouclier tibial antérieur sensiblement vertical, n'est cependant pas optimale, car à l'usage on se rend compte que lorsque le tibia est ostéoporotique, une fixation de l'implant tibial par impaction, comme dans les exemples qui ont été décrits, n'est pas suffisante pour obtenir une bonne stabilité de l'implant tibial, en particulier pour éviter des rotations autour de l'axe médio-latéral de celui-ci qui le feraient basculer vers l'avant ou vers l'arrière. L'addition de vis, traversant le bouclier tibial vertical pour s'ancrer sur cet os insuffisamment sain, ne constituerait pas un remède satisfaisant à cette situation.

Le but de l'invention est de proposer une amélioration des prothèses de cheville en trois éléments connues qui puisse être utilisée sur des patients victimes d'ostéoporose, en particulier au niveau de leur tibia, avec une excellente stabilité.

A cet effet, l'invention a pour objet une prothèse de cheville comportant un implant tibial, un implant talien et un insert sur lequel s'articulent lesdits implants, selon laquelle :
- la face supérieure de l'implant tibial comporte au moins une ailette d'ancrage de l'implant tibial dans le tibia du patient, et la face inférieure de l'implant tibial est une surface d'articulation avec la surface supérieure de l'insert ;
- la face inférieure de l'implant talien comporte des moyens d'ancrage sur l'os talien du patient, et la face supérieure de l'implant talien est une surface d'articulation avec la surface inférieure de l'insert, ladite surface d'articulation étant en forme de selle et comportant un sillon central longitudinal dans lequel l'insert peut se déplacer longitudinalement et tourner transversalement ;
caractérisée en ce que la face supérieure de l'implant tibial comporte sur son bord antérieur un bouclier destiné à couvrir la partie inférieure antérieure du tibia du patient, et dont la face tibiale est inclinée et forme un angle (α) avec l'horizontale compris entre 15° et 45°, et en ce que, dans ce bouclier, sont ménagés des passages pour des vis osseuses qui traversent le bouclier de façon ascendante et débouchent sur sa face tibiale inclinée.

La face inférieure de l'implant tibial peut être convexe et en portion de sphère et présenter un rayon de courbure, et la surface supérieure de l'insert est alors concave en portion de sphère et présente un rayon de courbure égal à celui de la face inférieure de l'implant tibial.

La face inférieure de l'implant tibial peut être convexe et présenter dans le plan antério-postérieur de la prothèse un premier rayon de courbure et, dans le plan médio-latéral de la prothèse, un deuxième rayon de courbure, et en la surface supérieure de l'insert est alors concave et présente les mêmes rayons de courbure que la face inférieure de l'implant tibial, la différence entre lesdits premier et deuxième rayons de courbure étant comprise entre 1 et 5% dudit deuxième rayon de courbure.

Les moyens d'ancrage de l'implant talien peuvent comporter au moins une ailette transversale inclinée par rapport à la surface inférieure de l'implant talien d'un angle (β) orienté vers l'arrière de la prothèse et dont la valeur est comprise entre 20 et 70°, de préférence entre 30 et 50°.

L'implant talien peut alors comporter au moins deux ailettes transversales disposées côte à côte.

La largeur de l'ailette transversale ou la somme des largeurs des ailettes transversales placées côte à côte peut représenter entre 40 et 80% de la largeur de l'implant talien.

L'au moins une ailette d'ancrage de l'implant tibial dans le tibia du patient peut être en forme générale de triangle rectangle, dont l'hypoténuse repose sur la face supérieure de l'implant tibial et dont le petit côté est orienté vers l'avant de l'implant tibial.

La longueur de l'hypoténuse de l'ailette tibiale peut représenter entre 50% et 80% de la longueur totale de la face supérieure de l'implant tibial.

L'angle supérieur de l'ailette tibiale peut être compris entre 80° et 120°.

L'au moins une ailette d'ancrage de l'implant tibial et les moyens d'ancrage de l'implant talien n'ont, de préférence, pas subi de traitement de surface destiné à favoriser la croissance osseuse, alors que d'autres portions desdits implants destinées à être en contact avec le tibia ou l'os talien ont de préférence subi un tel traitement de surface.

L'invention a également pour objet un kit comprenant une pluralité de prothèses de cheville du type précédent, caractérisé en ce que lesdites prothèses ont des dimensions différentes mais présentent toutes sur les faces inférieures de leurs implants tibiaux et sur les faces supérieures de leurs inserts des rayons de courbure identiques.

L'invention a également pour objet un kit comportant au moins une prothèse de cheville du type précédent, caractérisé en ce qu'il comporte également un guide de coupe pour la préparation du tibia en vue de la réception du bouclier d'un implant tibial du type précédent.

Il peut comporter également un guide de coupe pour la préparation du tibia en vue de la réception du bouclier d'un implant tibial du type précédent.

Comme on l'aura compris, l'invention repose sur la présence sur l'implant tibial d'un bouclier tibial antérieur qui présente deux caractéristiques essentielles le distinguant du bouclier tibial antérieur vertical connu dans EP-A-1 915 975.

La première de ces caractéristiques est que la face postérieure du bouclier (celle qui est au contact du tibia) ne forme pas un angle droit avec la surface supérieure horizontale de l'implant tibial, mais forme avec elle un angle de l'ordre de 30°, plus précisément entre 15° et 45°. Avant l'implantation de la prothèse, l'extrémité du tibia du patient est conformée par le chirurgien pour pouvoir être en contact avec le bouclier sur toute la surface de celui-ci, y compris la surface postérieure inclinée à 15-45°. Cette configuration garantit qu'on n'aura pas le basculement de l'implant vers l'avant ou l'arrière tel qu'on l'a décrit dans le cas de EP-A-1 915 975.

A cet effet, et c'est la seconde des caractéristiques distinctives principales, on complète la fixation de l'implant tibial par des vis osseuses qui traversent le bouclier incliné en étant orientées obliquement vers le haut, et viennent s'ancrer dans le tibia.

L'usage de telles vis, ancrées horizontalement dans le tibia, est aussi envisagé dans le cas des implants à bouclier vertical de l'art antérieur, mais si elles ne sont pas posées correctement, et/ou sont posées sur un os de qualité dégradée par une ostéoporose elles n'évitent pas les basculements de l'implant. L'invention permet de sécuriser la pose et le maintien en place de l'implant tibial, même sur un os ostéoporotique, et donc de fiabiliser dans le temps l'ensemble du fonctionnement de la prothèse de cheville.

Le fait que le bouclier ne couvre pas la face antérieure sensiblement verticale du tibia, mais seulement une partie rendue oblique de son extrémité inférieure, fait qu'il supporte une partie de l'effort sensiblement vertical qui s'exerce sur cette extrémité inférieure, et c'est aussi ce qui aide à son maintien en place lors de l'utilisation de la prothèse.

La conformation de l'extrémité inférieure du tibia qui doit être réalisée avant la mise en place de l'implant tibial comporte, en plus de la coupe horizontale nécessaire également pour les implants tibiaux classiques, une coupe antérieure de l'extrémité du tibia pour former un plan dont l'inclinaison correspond à celle du bouclier. Mais cela ne représente pas un réel inconvénient. Le chirurgien peut, à cet effet, s'aider d'un guide de coupe adéquat, et effectuer les deux coupes dans le même mouvement. Le temps de pose de la prothèse et la difficulté de cette pose ne s'en trouvent donc pas sensiblement affectés.

L'invention sera mieux comprise à la lecture de la description qui suit, donnée en référence aux figures annexées suivantes :
- La figure 1 qui montre un exemple de prothèse de cheville selon l'invention, vue en perspective ;
- La figure 2 qui montre ce même exemple vu de profil ;
- La figure 3 qui montre vu de dessus en perspective l'implant tibial des figures 1 et 2 ;
- La figure 4 qui montre vu de face ce même implant tibial ;
- La figure 5 qui montre une variante de ce même implant tibial, vue en perspective postérieure ;
- La figure 6 qui montre en perspective latérale un guide de coupe pour la conformation du tibia en vue de la mise en place d'un implant tibial de la prothèse de cheville selon l'invention ;
- La figure 7 qui montre en perspective vu de dessus le plateau de ce guide de coupe ;
- La figure 8 qui montre en perspective vu de dessous ce même plateau ;
- La figure 9 qui montre en vue de face la plaque de ce guide de coupe.

Il doit être entendu que l'exemple de mise en œuvre de l'invention qui va être décrit ne constitue qu'un exemple non limitatif, qui en plus du bouclier antérieur incliné de 30° environ par rapport à l'horizontale qui est le cœur de l'invention, présente un certain nombre d'autres caractéristiques avantageuses qui pourront être combinées les unes aux autres de diverses façons, au choix du fabricant de la prothèse et des souhaits de ses clients, comme cela sera commenté par la suite.

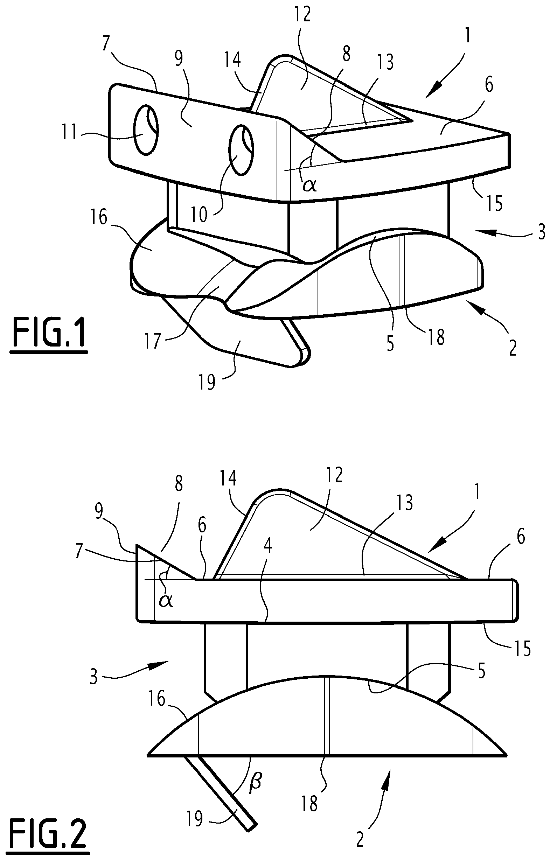

Les figures 1 et 2 montrent les trois éléments d'un exemple de prothèse selon l'invention, assemblés les uns sur les autres dans leurs positions relatives habituelle qu'ils sont censés occuper lors que la prothèse est implantée et que la cheville du patient est au repos.

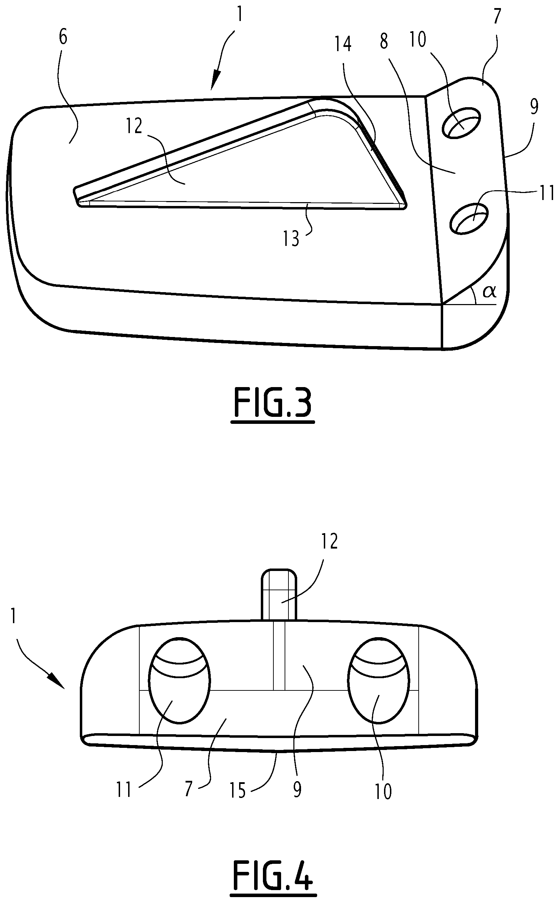

Le premier élément de la prothèse est un implant tibial 1 ancré sur l'extrémité inférieure du tibia du patient préalablement adéquatement conformée, et, que l'on voit isolément sur les figures 3, 4 et 5.

Le deuxième élément de la prothèse est un implant talien 2 ancré sur la face supérieure de l'os talien préalablement adéquatement conformée.

Le troisième élément de la prothèse est un insert 3, présentant une surface supérieure 4 sur laquelle s'articule l'implant tibial 1, et une surface inférieure 5 sur laquelle s'articule l'implant talien 2.

On va d'abord décrire l'implant tibial 1.

Il présente des dimensions extérieures qui le rendent adapté à une fixation à l'extrémité inférieure du tibia du patient, préalablement conformée à cet effet, comme cela est classique.

Sa face supérieure 6 est, de façon générale, plane et rectangulaire, mais elle présente deux éléments particuliers.

Selon l'invention, elle présente sur son bord antérieur, qui correspond au bord antérieur du tibia du patient, un bouclier 7 qui est destiné à couvrir la partie inférieure antérieure du tibia du patient. Contrairement à ce qui se passe dans le document EP-A-1 915 975 déjà cité, ce bouclier n'est pas vertical, mais a une face tibiale de contact 8 avec le tibia qui forme un angle α avec l'horizontale (autrement dit le prolongement de la face supérieure 6 de l'implant), cet angle α étant ouvert en direction de l'avant de la prothèse et étant compris entre 15 et 45°, typiquement égal à 30°. La face antérieure 9 du bouclier 7, c'est-à-dire la face opposée à cette face tibiale inclinée 8, est verticale dans l'exemple représenté, comme cela est préférable sans, toutefois, être obligatoire. Une telle face antérieure 9 verticale augmente l'épaisseur du bouclier par rapport à une surface antérieure 9 qui serait oblique, donc augmente sa rigidité, et est favorable aussi pour l'efficacité des vis d'ancrage.

A travers cette face antérieure 9 sont ménagés deux passages 10, 11 pour le passage de vis osseuses (non représentées). Ces passages 10, 11 traversent le bouclier 7 selon une direction ascendante, formant de préférence un angle de 60° à 80° avec l'horizontale.

Les vis osseuses débouchent sur la face tibiale inclinée 8 du bouclier 7, et viennent se loger dans le tibia du patient en assurant une bonne solidarisation de l'implant tibial 1 au tibia. Il n'est pas nécessaire que les vis osseuses débouchent perpendiculairement à la face postérieure 8 du bouclier 7, donc ledit angle que les passages 10, 11 forment avec l'horizontale n'est pas dépendant de la valeur dudit angle α.

L'angulation α à 15-45° du bouclier 7, selon l'invention, assure que l'implant tibial 1 ne subira pas de basculement d'avant en arrière ou inversement lors de son utilisation, et qu'il demeurera parfaitement horizontal, pour peu que la découpe préalable du tibia ait été correctement faite. Cette découpe est soumise aux mêmes contraintes que pour l'implant de EP-A-1 915 975, auxquelles s'ajoute la conformation de la surface du tibia sur laquelle la face tibiale inclinée 8 du bouclier 7 vient se plaquer. Mais cette conformation est réalisée par un guide de coupe adéquat, dans le même mouvement que la conformation de la face inférieure du tibia sur laquelle va reposer la face supérieure 6 de l'implant tibial 1. Un tel exemple de guide de coupe sera décrit plus loin.

Cette solidarisation de l'implant tibial 1 au tibia est aussi assurée par une ailette 12 disposée sur la face supérieure 6 de l'implant 1 et qui s'étend, de préférence sur la majeure partie de l'axe longitudinal de ladite face 6 (c'est-à-dire de l'axe qui va de la partie postérieure à la partie antérieure de l'implant). Cette ailette est en forme générale de triangle rectangle et doit s'insérer dans le tibia du patient.

L'emploi d'ailettes approximativement en triangle rectangle sur un implant tibial est déjà connu dans l'art antérieur. Ces ailettes connues ont cependant une forme sensiblement différente de celle que l'invention comporte de façon préférée. Habituellement, les ailettes approximativement en triangle rectangle ont le petit côté du triangle qui repose sur la surface supérieure de l'implant tibial, et leur hypoténuse (qui peut être, en fait, légèrement courbée) est tournée vers l'avant du tibia. Elles ont donc une hauteur relativement importante et viennent s'insérer dans la cavité médullaire du tibia, préalablement aménagée à cet effet.

L'ailette 12 telle que représentée, selon une variante préférée de l'invention, a au contraire son hypoténuse 13 qui repose sur la face supérieure 6 de l'implant tibial 1. Le petit côté 14 de l'ailette est orienté vers l'avant de l'implant tibial 1, donc en direction du bouclier 7.

Par rapport à la configuration classique des ailettes en triangle rectangle de grande hauteur, la configuration selon une variante préférée de l'invention qui vient d'être décrite présente divers avantages.

Du fait de ses plus faibles dimensions, cette forme 12 d'ailette nécessite d'enlever moins d'os sain que les ailettes classiques, qui sont plus étroites mais ont une grande hauteur. Le tibia est donc moins fragilisé, ce qui est important surtout si le patient souffre d'ostéoporose. Les ailettes classiques sont davantage susceptibles d'aboutir à la formation indésirable de géodes osseuses. Egalement, le fait d'avoir l'hypoténuse 13 placée sur la surface supérieure 6 de l'implant tibial 1, avec le petit côté 14 de l'ailette dirigé vers l'avant du tibia, permet d'avoir à la fois une relativement faible hauteur de l'ailette 12 et une surface de contact importante de l'ailette 12 avec la surface supérieure 6 de l'implant tibial 1, d'où une bonne répartition des efforts exercés sur l'implant tibial 1 lors de la marche, ce qui est favorable à une bonne tenue dans le temps de l'implant tibial 1.

On considère que, préférentiellement, la longueur de l'hypoténuse 13 de l'ailette 12 représente 50% à 80%, typiquement 70%, de la longueur totale de la face supérieure 6 de l'implant tibial 1, ladite longueur totale incluant donc la portion sur laquelle le bouclier 8 est présent.

Préférentiellement, la hauteur de l'ailette 12 représente 60% à 80% de la hauteur totale de l'implant tibial 1, typiquement les 2/3 de cette hauteur.

Comme on l'a dit, l'ailette 12 a une forme générale de triangle rectangle, mais l'angle supérieur qui, comme représenté, peut être arrondi, n'est pas forcément rigoureusement égal à 90°, et peut se situer dans une gamme de 80° à 120°.

Préférentiellement, l'ailette 12 présente une largeur comprise entre 2 et 3 mm, par exemple 2,5 mm environ. Il y a un compromis à trouver entre une épaisseur faible qui ne nécessite pas d'enlever beaucoup d'os sain et une épaisseur élevée qui améliore la solidité de l'ancrage, si l'os peut le supporter. Dans la variante représentée, un espace est conservé entre l'extrémité antérieure de la base de l'ailette 12 et la base du bouclier 8. Mais ce n'est qu'une variante d'exécution, et il demeurerait conforme à l'invention de faire buter la base de l'ailette 12 contre la base du bouclier 8.

On peut envisager la présence de plusieurs ailettes 12 parallèles au lieu d'une seule comme dans l'exemple représenté.

Concernant la surface inférieure 15 de l'implant tibial 1, elle doit être capable de procurer une possibilité d'articulation avec la surface supérieure correspondante 4 de l'insert 3, de façon à contribuer à restituer ses mouvements naturels à la cheville du patient. On peut ainsi conférer à cette surface inférieure 15 une forme convexe sensiblement en portion de sphère, et ayant un rayon de courbure R sensiblement identique à celui que présente la surface supérieure 4 de l'insert 3, celle-ci étant le « négatif » de la surface inférieure 15 de l'implant tibial 1, et étant donc concave en portion de sphère, de façon à articuler l'implant tibial 1 et l'insert 3 l'un sur l'autre à la manière d'une rotule. Typiquement, ce rayon R est de l'ordre de 300 à 310 mm.

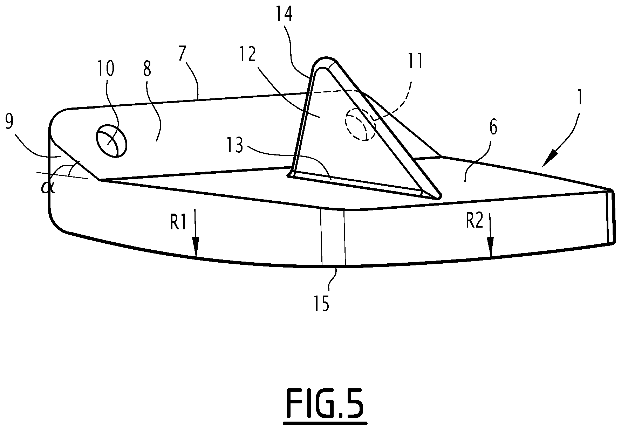

Une autre possibilité serait que cette surface inférieure 15 de l'implant tibial 1 soit convexe et ne présente pas un rayon de courbure unique, mais, comme représenté sur la figure 5 qui met cette configuration en évidence, deux rayons de courbure différents : un rayon de courbure R1 dans le plan antéro-postérieur et un autre rayon de courbure R2 dans le plan médio-latéral. R2 est supérieur à R1 dans l'exemple représenté, mais l'inverse serait aussi envisageable. Les différences entre ces deux rayons R1 et R2 doivent cependant rester faibles pour assurer un bon fonctionnement de l'articulation, de l'ordre de 1 à 5%, ce pourcentage étant calculé sur la base de R2.

Dans cette variante, on retrouverait de préférence sensiblement à l'identique cette configuration à deux rayons de courbure sur la surface supérieure 4 de l'insert 3.

Typiquement R1 peut être de 305 mm et R2 de 310 mm.

De manière générale, il est préférable d'avoir une congruence des surfaces de l'implant tibial 1 et de l'insert 3 qui sont au contact l'une de l'autre (autrement dit des rayons de courbure sensiblement identiques), de façon à avoir une bonne répartition des efforts sur l'ensemble de ces surfaces, en particulier quand l'articulation est dans sa position nominale où le contact est maximal entre l'implant tibial 1 et l'insert 3.

Il est d'usage de recouvrir les différents éléments d'une prothèse de cheville d'hydroxyapatite, ou d'une autre substance favorisant la croissance osseuse, dans les zones où les prothèses sont au contact de l'os du patient. Un autre type de traitement de surface, notamment un grenaillage, en plus ou à la place de ce revêtement, peut aussi contribuer à cette fonction. Cependant, on constate souvent que des géodes osseuses peuvent se former dans les zones revêtues lorsque celle-ci ont des formes un peu complexes. Les inventeurs jugent donc préférable de ne pas disposer de tel revêtement sur l'ailette 12 et, de manière générale de ne pas traiter sa surface par des moyens physiques ou chimiques. Le traitement chimique et/ou physique de la surface peut se limiter à tout ou partie de la face supérieure 6 de l'implant tibial 1 et aussi à celle du bouclier 8, ou de manière générale à une partie quelconque de l'implant tibial 1 destinée à venir au contact du tibia du patient, autre que l'ailette 12.

On va à présent décrire plus en détail l'implant talien 2.

Il peut avoir une configuration générale conforme à celle des implants taliens connus, par exemple dans les documents cités en introduction. Mais selon une variante préférée de l'invention, il peut avoir la configuration représentée sur les figures. Celle-ci, comme c'est le cas dans la prothèse de WO-A-00/69373, présente une surface supérieure 16 en forme de selle, avec un sillon central longitudinal 17 dans lequel l'insert 3 peut se déplacer longitudinalement et tourner latéralement. De cette façon, les surfaces d'articulation entre l'implant talien 2 et l'insert 3 ont des formes qui se correspondent, et l'implant talien 2 et l'insert 3 peuvent glisser l'un sur l'autre librement dans le plan sagittal, mais pas dans le plan frontal autrement qu'en rotation, ce qui a pour but de maintenir la prothèse en place et d'éviter la dislocation en cas d'hyperlaxité latérale de la cheville du patient. La section transversale du sillon 17 présente un rayon de courbure et le sillon 17 sépare la surface supérieure 16 de l'implant talien 2 en deux portions connectées l'une à l'autre par des lignes d'inflexion selon lesquelles la direction de courbure s'inverse. Chacune de ces portions a une forme en portion de sphère.

Mais cette configuration préférée de l'implant talien 2 se distingue par deux particularités.

En premier lieu, sa surface inférieure 18 n'est pas courbe, mais plane. Cela simplifie la conformation de l'os talien, par rapport au cas classique où cette surface inférieure est courbe et reproduit approximativement la forme nominale de l'os talien. Même dans les implants taliens à surface inférieure courbe, une conformation de la surface de l'os talien pour l'adapter à l'implant est de toute façon nécessaire, de sorte que l'implantation de l'implant talien selon cette variante préférée de l'invention ne nécessite pas d'étape supplémentaire par rapport à la pratique la plus courante. Et même, la surface inférieure 18 plane de cette variante de l'invention simplifie cette conformation de l'os talien.

D'autre part, l'ancrage de l'implant talien 2 dans l'os talien est assuré non plus par des picots sensiblement cylindriques, mais par une ailette talienne transversale plane 19 inclinée par rapport à la surface inférieure 18 de l'implant talien 2 d'un angle β orienté vers l'arrière de la prothèse et dont la valeur est comprise entre 20 et 70°, par exemple 45° comme représenté, de préférence entre 30° et 50° pour faciliter l'insertion de l'ailette 19 dans l'os talien. L'encombrement vertical de l'ailette 19 est typiquement de 4 à 10 mm, selon la taille globale de la prothèse qui dépend, bien sûr, des mensurations du patient. La largeur de l'ailette talienne 19 correspond typiquement à 40 à 80% de la largeur totale de l'implant talien. L'épaisseur de l'ailette talienne 19 est typiquement de l'ordre de 2,5 mm.

En variante, on pourrait avoir au moins deux telles ailettes 19 disposées côte à côte et/ou les unes derrière les autres, quoique multiplier les éléments d'ancrage tendrait à augmenter les risques de formation de géodes osseuses. Dans le cas d'ailettes multiples disposées côte à côte, c'est la somme de leurs largeurs qui devrait représenter typiquement entre 40 et 80% de la largeur totale de l'implant talien.

La présence d'au moins une ailette transversale 19 telle qu'on vient de la décrire permet de concilier :
- un très bon ancrage de l'implant talien 2 sur l'os talien ;
- et la stricte planéité de la surface inférieure 18 de l'implant talien, qui procure une surface de contact avec l'os talien moindre que dans les cas connus où cette surface suit approximativement la courbure naturelle de l'os talien.

Les inventeurs recommandent de ne pas traiter la surface de l'ailette transversale talienne 19 par dépôt d'hydroxyapatite (ou équivalent) et/ou grenaillage, pour les mêmes raisons que celles citées à propos l'ailette tibiale longitudinale 12. Ce traitement, s'il est effectué, est donc, de préférence, limité à tout ou partie de la surface inférieure plane 18 de l'implant talien 2 ou, de manière générale, à toute partie de l'implant talien 2 destinée à venir au contact de l'os talien, autre que l'ailette transversale 19.

On va à présent décrire l'insert 3.

Il présente sur sa face supérieure 4 et sa face inférieure 5 les caractéristiques de forme et de dimensions qui résultent des conformations respectives de la face inférieure 15 de l'implant tibial 1 et de la face supérieure 16 de l'implant talien 2 telles qu'on vient de les décrire. Ainsi, la face supérieure 4 est creusée avec un ou des rayons de courbure correspondant sensiblement à celui ou ceux de la face inférieure 15 de l'implant tibial, et la face inférieure 5 présente une forme de selle qui est sensiblement le « négatif » de la forme en selle de la surface supérieure 16 de l'implant talien 2. On retrouve donc sensiblement, de ce point de vue, la configuration représentée dans le document WO-A-00/69373 déjà cité.

A titre d'exemple non limitatif, les dimensions principales d'une prothèse de cheville selon l'invention, dans sa variante préférée représentée, seraient les suivantes.

L'implant tibial 1 a une forme sensiblement rectangulaire, ou une forme légèrement trapézoïdale pour laquelle le grand côté étant le bord antérieur de l'implant. Sa longueur est de 40,5 mm et sa largeur maximale 32 mm, Sa hauteur totale, ailette 12 comprise, est de 15 mm. Son épaisseur, hors l'ailette 12 et le bouclier 7, est de 5 mm environ (elle varie du fait de la présence des rayons de courbure R1 et R2 de sa face inférieure 15). La hauteur du bouclier 7 est de 10,1 mm et sa longueur est de 10 mm. L'angle α est de 30° Les rayons R1 et R2 de la face inférieure 15 sont respectivement de 310 et 315 mm. L'ailette 12 a un angle au sommet de 90° et une hypoténuse de longueur 27 mm.

L'implant talien 2 a une forme générale rectangulaire ou trapézoïdale, quoique, comme représenté sur la figure 1, ses bords antérieur et postérieurs peuvent présenter chacun une échancrure au niveau du débouché du sillon 17. Sa longueur est de 38 mm et sa largeur maximale de 29,5 mm. Sa hauteur maximale est de 17 mm ailette 19 comprise et de 9 mm hors ailette, et le rayon de courbure de sa face supérieure 16 est de 26,3 mm. Sa hauteur minimale hors ailette 17 (mesurée au fond du sillon 17) est de 5,4 mm. Les rayons de courbure de la face supérieure 16 sont de 29,4 mm au niveau du sillon 17 et de 19,5 mm de part et d'autre du sillon 17. L'ailette 19 forme un angle β de 45° avec la face inférieure 18 de l'implant talien 2, elle est placée à 6 mm dur bord antérieur de l'implant talien 2 et s'étend sur une hauteur de 7,7 mm.

L'insert 3 a une forme générale rectangulaire ou trapézoïdale. Sa longueur est de 26 mm et sa largeur maximale est de 28 mm. Si sa forme est trapézoïdale, sa largeur minimale est de 24 mm. Son épaisseur totale maximale est de 10,6 mm, et son épaisseur, si on ne tient pas compte de la partie destinée à épouser la surface supérieure 16 de l'implant talien et à pénétrer dans le sillon 17 varie entre 5,3 mm (dans la partie centrale de l'insert 3) et 8,1 mm (aux bords antérieur et postérieur de l'insert 3). La face supérieure 4 de l'insert 3 présente des courbures qui reproduisent « en négatif » celles de la face inférieure 15 de l'implant tibial 1 que l'on a données.

Bien entendu, les dimensions de la prothèse selon l'invention sont à adapter à la morphologie précise du patient, et peuvent être choisies, notamment, à partir de l'exemple précédent en modifiant lesdites dimensions de façon homothétique.

Une façon particulièrement avantageuse de mettre en œuvre l'invention consiste à la proposer sous la forme d'un kit comprenant une série de prothèses dont les dimensions générales seraient différentes et susceptibles de convenir à des patients de diverses morphologies. Toutefois, il est avantageux que toutes les prothèses du kit présentent sur les faces inférieures 15 de leurs implants tibiaux 1 et sur les faces supérieures 4 de leurs inserts 3 le même rayon de courbure R (ou les mêmes rayons de courbure R1 et R2 pour le cas de la figure 5). De cette façon, le chirurgien peut choisir, parmi les éléments des diverses prothèses du kit, ceux qui lui semblent les mieux adaptés à la morphologie précise du patient après les découpes du tibia et de l'os talien, en étant assuré que deux éléments pris dans deux prothèses différentes seront néanmoins compatibles.

Le kit peut aussi comporter un ou plusieurs guides et outils de coupe spécialement conçus pour configurer l'extrémité inférieure du tibia de manière à y permettre la mise en place de l'implant tibial 1 selon l'invention avec son bouclier 7. Il peut aussi comporter un ou plusieurs autres guides et outils de coupe conçus pour configurer la surface supérieure de l'os talien, de manière à y permettre la mise en place de l'implant talien 2 à surface inférieure 18 plane et à ailette inclinée 19 figurant dans le kit.

La découpe de l'extrémité inférieure du tibia du patient peut être effectuée de la façon suivante.

Dans un premier temps, on réalise, à ladite extrémité inférieure, la découpe d'une surface plane et sensiblement perpendiculaire à l'axe longitudinal du tibia. Cela peut être exécuté à l'aide d'un guide de découpe et d'une lame de découpe connus classiquement à cet effet pour réaliser la mise en place d'implants tibiaux classiques dépourvus de bouclier incliné, comme par exemple celui décrit dans EP-A-1 915 975 déjà cité, qui comporte une butée transversale verticale venant en appui sur la face antérieure du tibia.

Dans un deuxième temps, on réalise une découpe de la partie antérieure inférieure du tibia de façon à y ménager :
- Une surface inclinée sur laquelle la face postérieure inclinée 8 du bouclier 7 vient s'appuyer lors de la mise en place de l'implant tibial 1 selon l'invention ;
- Et, si ledit implant tibial 1 comporte, comme cela est préféré, une ou plusieurs ailettes 12, une ou des fentes dans la ou lesquelles la ou lesdites ailettes 12 viendra/viendront s'insérer lors de cette mise en place.

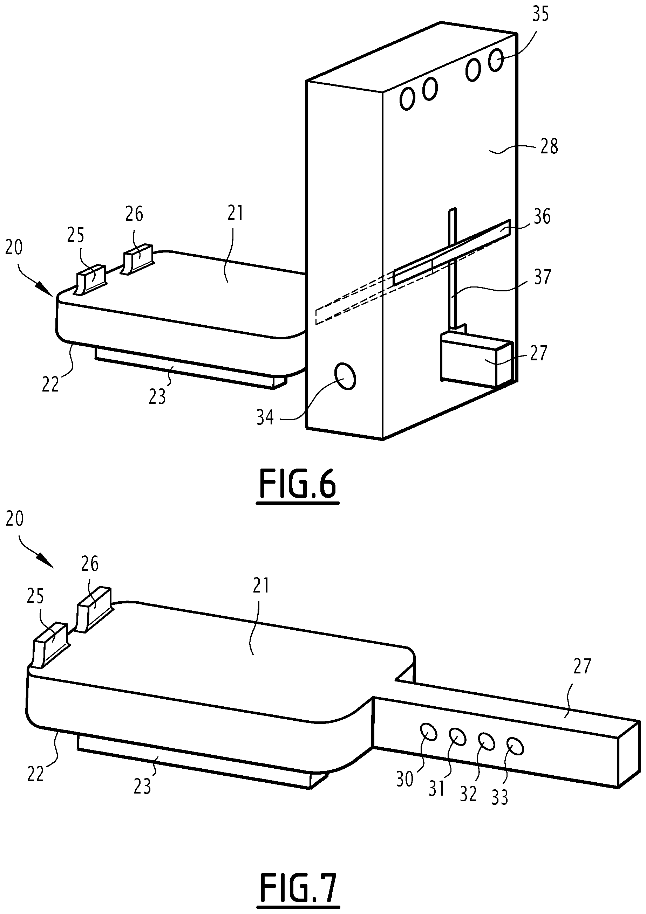

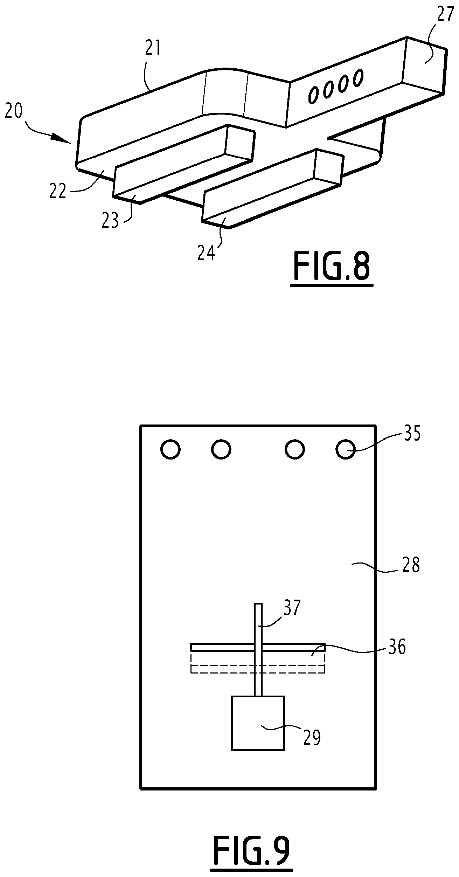

A cet effet, on peut utiliser, de façon non limitative, le guide de coupe représenté sur les figures 6 à 9.

Il comporte un plateau 20 dont la surface supérieure 21 est conçue pour venir en appui sur la surface plane et sensiblement perpendiculaire à l'axe longitudinal du tibia qui a été découpée lors de la première étape de la découpe, et dont la surface inférieure 22 comporte, dans l'exemple représenté, deux rails 23, 24 qui permettent au plateau de coulisser et de s'appuyer sur une entretoise rainurée disposée sur l'os talien, et qui a pour fonction d'imposer la tension ligamentaire qui sera désirée après la pose de la prothèse, selon son épaisseur conjuguée à celle du plateau 20. On applique à cette effet une technique opératoire dite « AES » très comparable à celle décrite dans le document EP-B-1 301 148 qui a pour objet une prothèse de cheville en trois parties dont l'implant tibial, d'une part, est dépourvu du bouclier selon la présente invention, et, d'autre part, comporte une ailette en triangle rectangle de grande hauteur dont l'hypoténuse est tournée vers l'avant du tibia, contrairement à une variante préférée de la présente invention.

Le bord postérieur de la face supérieure 21 du plateau 20 comporte des moyens d'assistance à la mise en position et au maintien du plateau 20 qui, dans l'exemple représenté, sont constitués par deux butées 25, 26 que l'on fait venir en appui contre la corticale externe arrière du tibia. Sur le bord antérieur du plateau 20 se trouve un appendice longitudinal 27 de section sensiblement carrée dans l'exemple représenté.

Cet appendice 27 est destiné à coopérer avec une plaque 28 qui comporte un orifice traversant 29 débouchant sur les faces antérieure et postérieure de la plaque 28, et dans lequel l'appendice 27 peut coulisser avec un minimum de jeu. En particulier, il faut que l'angle formé par la plaque 28 et le plateau 20 soit bien déterminé et de valeur permanente (généralement 90°). Il est donc déconseillé de conférer à cet appendice 27 une section circulaire qui lui permettrait aisément de tourner à l'intérieur de l'orifice 29 lors de la mise en place et de l'utilisation du guide de coupe.

La position de la plaque 28 le long de l'appendice 27 peut être fixée, comme représenté, au moyen d'orifices transversaux 30, 31, 32, 33 ménagés dans l'appendice 27 et d'un orifice transversal 34 ménagé dans la plaque 28 et traversant l"orifice longitudinal 29. Une goupille (non représentée) ou tout autre dispositif de blocage fonctionnellement analogue peut ainsi être logée dans l'orifice transversal 34 de la plaque 28 en traversant l'un des orifices transversaux 30-33 de l'appendice 27. On peut ainsi adapter la géométrie de l'ensemble plateau 20-plaque 28 à celles du tibia du patient et de l'implant tibial 1 qui sera implanté.

Une fois cette position relative de la plaque 28 et du plateau 20 déterminée et assurée par le dispositif de blocage, on assure la fixation du guide de coupe au tibia, par exemple au moyen de clous traversant des orifices longitudinaux 35 situés dans la partie supérieure de la plaque 28.

Puis on réalise la découpe de l'extrémité antérieure basse du tibia, de façon à permettre à la face postérieure inclinée 8 du bouclier 7 de l'implant tibial 1 de venir s'y placer. A cet effet, la plaque 28 comporte une fente transversale 36 qui s'étend entre la face antérieure et la face postérieure de la plaque 28, et qui est inclinée selon un angle égal à l'angle α par rapport à l'horizontale. Cette fente 36 présente une largeur et une épaisseur suffisantes pour laisser passer et guider une lame de coupe de type classique que le chirurgien pourra y introduire, afin de réaliser la découpe du tibia pour la réception du bouclier 7.

Si l'implant tibial à poser comporte une seule ailette 12, la plaque 28 comporte également une fente verticale 37 située dans le plan médian transversal de la plaque 28. Cette fente 37 est dimensionnée pour permettre l'introduction d'une lame de coupe pour ménager dans le tibia un orifice dans lequel l'ailette 12 pourra s'insérer lors de la mise en place de l'implant tibial 1. Si plusieurs ailettes 12 sont prévues sur l'implant tibial 1, il faudra, bien sûr, prévoir un nombre semblable de fentes 37 à des emplacements correspondants à ceux des ailettes 12.

Avant ou après la découpe du tibia telle qu'on vient de la décrire, le chirurgien procède à la découpe de l'os talien qui va lui permettre de recevoir un implant talien 2 selon l'invention. En particulier, cet outil de coupe doit pouvoir ménager une surface supérieure plane sur l'os talien, si la surface inférieure 18 de l'implant talien 2 est plane, comme dans l'exemple préféré représenté. Cela peut être réalisé, par exemple, à l'aide d'un guide de coupe comparable à celui décrit dans le document WO-A-01/89427.

Comme on l'a dit, ces guides de coupe ou au moins certains d'entre eux, notamment celui destiné à la préparation du tibia à la réception de l'implant tibial 1 selon l'invention et de son bouclier 7 qui est spécifique à l'implantation de la prothèse de cheville selon l'invention, peuvent avantageusement être inclus dans un kit incluant les éléments de la prothèse de cheville. Ce kit peut également inclure des outils de coupe et des moyens de fixation des guides de coupe sur les os correspondants du patient, surtout si ces outils de coupe et moyens de fixation ne sont pas de types couramment disponibles isolément auprès des chirurgiens. Il peut aussi inclure tout autre outil pour assister la mise en place de la prothèse selon l'invention, notamment des entretoises rainurées de diverses épaisseurs destinées à être utilisées conjointement avec le plateau 20 du guide de coupe des figure 6 à 9 (ou tout autre type de guide de coupe qui reposerait sur des principes comparables).

Une excellente stabilité de la prothèse est obtenue notamment lorsqu'on conjugue l'utilisation d'un implant tibial 1 à bouclier incliné selon l'invention et une absence de revêtement/traitement de surface sur les ailettes 12, 19 des implants 1, 2 qui minimise le risque de formation de géodes osseuses aux niveaux des ancrages des implants 1, 2.

## Revendications

1. Prothèse de cheville comportant un implant tibial (1), un implant talien (2) et un insert (3) sur lequel s'articulent lesdits implants (1, 2), selon laquelle :
- la face supérieure (6) de l'implant tibial (1) comporte au moins une ailette (12) d'ancrage de l'implant tibial (1) dans le tibia du patient, et la face inférieure (15) de l'implant tibial (1) est une surface d'articulation avec la surface supérieure (4) de l'insert (3) ;
- la face inférieure (18) de l'implant talien (2) comporte des moyens d'ancrage sur l'os talien du patient, et la face supérieure (16) de l'implant talien (2) est une surface d'articulation avec la surface inférieure (5) de l'insert (3), ladite surface d'articulation étant en forme de selle et comportant un sillon central longitudinal (17) dans lequel l'insert (3) peut se déplacer longitudinalement et tourner transversalement ;
**caractérisée en ce que** la face supérieure (6) de l'implant tibial (1) comporte sur son bord antérieur un bouclier (7) destiné à couvrir la partie inférieure antérieure du tibia du patient, et dont la face tibiale (8) est inclinée et forme un angle (α) avec l'horizontale compris entre 15° et 45°, et **en ce que**, dans ce bouclier (7), sont ménagés des passages (10, 11) pour des vis osseuses qui traversent le bouclier (7) de façon ascendante et débouchent sur sa face tibiale inclinée (8).

2. Prothèse de cheville selon la revendication 1, **caractérisée en ce que** la face inférieure (15) de l'implant tibial (1) est convexe et en portion de sphère et présente un rayon de courbure (R), et **en ce que** la surface supérieure (4) de l'insert (3) est concave en portion de sphère et présente un rayon de courbure égal à celui (R) de la face inférieure (15) de l'implant tibial (1).

3. Prothèse de cheville selon la revendication 1, **caractérisée en ce que** la face inférieure (15) de l'implant tibial (1) est convexe et présente dans le plan antério-postérieur de la prothèse un premier rayon de courbure (R1) et, dans le plan médio-latéral de la prothèse, un deuxième rayon de courbure (R2), et **en ce que** la surface supérieure (4) de l'insert (3) est concave et présente les mêmes rayons de courbure (R1, R2) que la face inférieure (15) de l'implant tibial (1), la différence entre lesdits premier (R1) et deuxième (R2) rayons de courbure étant comprise entre 1 et 5% dudit deuxième rayon de courbure (R2).

4. Prothèse de cheville selon l'une des revendications 1 à 3, **caractérisée en ce que** les moyens d'ancrage de l'implant talien comportent au moins une ailette transversale (19) inclinée par rapport à la surface inférieure (18) de l'implant talien (2) d'un angle (β) orienté vers l'arrière de la prothèse et dont la valeur est comprise entre 20 et 70°, de préférence entre 30 et 50°.

5. Prothèse de cheville selon la revendication 4, **caractérisée en ce que** son implant talien (2) comporte au moins deux ailettes transversales (19) disposées côte à côte.

6. Prothèse de cheville selon la revendication 4 ou 5, **caractérisée en ce que** la largeur de l'ailette transversale (19) ou la somme des largeurs des ailettes transversales (19) placées côte à côte représente entre 40 et 80% de la largeur de l'implant talien (2).

7. Prothèse de cheville selon l'une des revendications 1 à 6, **caractérisée en ce que** l'au moins une ailette (12) d'ancrage de l'implant tibial (1) dans le tibia du patient est en forme générale de triangle rectangle, dont l'hypoténuse (13) repose sur la face supérieure (6) de l'implant tibial (1) et dont le petit côté (14) est orienté vers l'avant de l'implant tibial (1).

8. Prothèse de cheville selon la revendication 7, **caractérisée en ce que** la longueur de l'hypoténuse (13) de l'ailette tibiale (12) représente entre 50% et 80% de la longueur totale de la face supérieure (6) de l'implant tibial (1).

9. Prothèse de cheville selon la revendication 7 ou 8, **caractérisée en ce que** l'angle supérieur de l'ailette tibiale (12) est compris entre 80° et 120°.

10. Prothèse de cheville selon l'une des revendications 1 à 9, **caractérisée en ce que** l'au moins une ailette d'ancrage (12) de l'implant tibial (1) et les moyens d'ancrage de l'implant talien (2) n'ont pas subi de traitement de surface destiné à favoriser la croissance osseuse, alors que d'autres portions desdits implants (1, 2) destinées à être en contact avec le tibia ou l'os talien ont subi un tel traitement de surface.

11. Kit comprenant une pluralité de prothèses de cheville selon l'une des revendications 1 à 10, **caractérisé en ce que** lesdites prothèses ont des dimensions différentes mais présentent toutes sur les faces inférieures (15) de leurs implants tibiaux (1) et sur les faces supérieures (4) de leurs inserts (3) des rayons de courbure (R ; R1, R2) identiques.

12. Kit comportant au moins une prothèse de cheville selon l'une des revendications 1 à 10, **caractérisé en ce qu'**il comporte également un guide de coupe pour la préparation du tibia en vue de la réception du bouclier (7) de l'implant tibial (1) selon la revendication 1.

13. Kit selon la revendication 11, **caractérisé en ce qu'**il comporte également un guide de coupe pour la préparation du tibia en vue de la réception du bouclier (7) de l'implant tibial (1) selon la revendication 1.

## Patentansprüche

1. Knöchelprothese, umfassend ein Tibiaimplantat (1), ein Talusimplantat (2) und einen Einsatz (3), an dem die Implantate (1, 2) angelenkt sind, wobei:
- die obere Fläche (6) des Tibiaimplantats (1) mindestens eine Rippe (12) zur Verankerung des Tibiaimplantats (1) in der Tibia des Patienten umfasst und die untere Fläche (15) des Tibiaimplantats (1) eine Gelenkfläche mit der oberen Fläche (4) des Einsatzes (3) ist;
- die untere Fläche (18) des Talusimplantats (2) Einrichtungen zur Verankerung an dem Talusknochen des Patienten umfasst, und die obere Fläche (16) des Talusimplantats (2) eine Gelenkfläche mit der unteren Fläche (5) des Einsatzes (3) ist, wobei die Gelenkfläche sattelförmig ist und eine mittlere Längsnut (17) umfasst, in der der Einsatz (3) in Längsrichtung verschiebbar und in Querrichtung drehbar ist;
**dadurch gekennzeichnet, dass** die obere Fläche (6) des Tibiaimplantats (1) an ihrem vorderen Rand eine Schale (7) aufweist, die dazu bestimmt ist, den unteren vorderen Abschnitt der Tibia des Patienten abzudecken und deren Tibiafläche (8) geneigt ist und mit der Horizontalen einen Winkel (α) zwischen 15° und 45° bildet, und dass in dieser Schale (7) Durchgänge (10, 11) für Knochenschrauben angeordnet sind, die die Schale (7) aufsteigend durchdringen und an ihrer geneigten Tibiafläche (8) münden.

2. Knöchelprothese nach Anspruch 1, **dadurch gekennzeichnet, dass** die untere Fläche (15) des Tibiaimplantats (1) konvex und teilweise kugelförmig ist und einen Krümmungsradius (R) aufweist, und dass die obere Fläche (4) des Einsatzes (3) konkav und teilweise kugelförmig ist und einen Krümmungsradius aufweist, der gleich dem Krümmungsradius (R) der unteren Fläche (15) des Tibiaimplantats (1) ist.

3. Knöchelprothese nach Anspruch 1, **dadurch gekennzeichnet, dass** die untere Fläche (15) des Tibiaimplantats (1) konvex ist und einen ersten Krümmungsradius (R1) in der anterior-posterioren Ebene der Prothese und einen zweiten Krümmungsradius (R2) in der medio-lateralen Ebene der Prothese aufweist, und dass die obere Fläche (4) des Einsatzes (3) konkav ist und die gleichen Krümmungsradien (R1, R2) wie die untere Fläche (15) des Tibiaimplantats (1) aufweist, wobei der Unterschied zwischen dem ersten (R1) und dem zweiten (R2) Krümmungsradius zwischen 1 und 5 % des zweiten Krümmungsradius (R2) ist.

4. Knöchelprothese nach einem der Ansprüche 1 bis 3, **dadurch gekennzeichnet, dass** die Einrichtungen zur Verankerung des Talusimplantats mindestens eine Querrippe (19) umfassen, die in Bezug auf die untere Fläche (18) des Talusimplantats (2) um einen Winkel (β) geneigt ist, der zu der Rückseite der Prothese gerichtet ist und dessen Wert zwischen 20 und 70°, vorzugsweise zwischen 30 und 50°, ist.

5. Knöchelprothese nach Anspruch 4, **dadurch gekennzeichnet, dass** ihr Talusimplantat (2) mindestens zwei nebeneinander angeordnete Querrippen (19) umfasst.

6. Knöchelprothese nach Anspruch 4 oder 5, **dadurch gekennzeichnet, dass** die Breite der Querrippe (19) oder die Summe der Breiten der nebeneinander angeordneten Querrippen (19) zwischen 40 und 80 % der Breite des Talusimplantats (2) ist.

7. Knöchelprothese nach einem der Ansprüche 1 bis 6, **dadurch gekennzeichnet, dass** die mindestens eine Rippe (12) zur Verankerung des Tibiaimplantats (1) in der Tibia des Patienten im Wesentlichen in der Form eines rechtwinkligen Dreiecks ist, dessen Hypotenuse (13) auf der oberen Fläche (6) des Tibiaimplantats (1) aufliegt und dessen kurze Seite (14) zu der Vorderseite des Tibiaimplantats (1) ausgerichtet ist.

8. Knöchelprothese nach Anspruch 7, **dadurch gekennzeichnet, dass** die Länge der Hypotenuse (13) der Tibiarippe (12) zwischen 50 % und 80 % der Gesamtlänge der oberen Fläche (6) des Tibiaimplantats (1) ist.

9. Knöchelprothese nach Anspruch 7 oder 8, **dadurch gekennzeichnet, dass** der obere Winkel der Tibiarippe (12) zwischen 80° und 120° ist.

10. Knöchelprothese nach einem der Ansprüche 1 bis 9, **dadurch gekennzeichnet, dass** die mindestens eine Verankerungsrippe (12) des Tibiaimplantats (1) und die Einrichtungen zur Verankerung des Talusimplantats (2) keiner Oberflächenbehandlung zur Förderung des Knochenwachstums unterzogen wurden, während andere Abschnitte der Implantate (1, 2), die dazu bestimmt sind, mit der Tibia oder dem Talarknochen in Kontakt zu sein, einer solchen Oberflächenbehandlung unterzogen wurden.

11. Kit, umfassend eine Vielzahl von Knöchelprothesen nach einem der Ansprüche 1 bis 10, **dadurch gekennzeichnet, dass** die Prothesen unterschiedliche Abmessungen aufweisen, aber alle identische Krümmungsradien (R; R1, R2) an den Unterseiten (15) ihrer Tibiaimplantate (1) und an den oberen Flächen (4) ihrer Einsätze (3) aufweisen.

12. Kit, umfassend mindestens eine Knöchelprothese nach einem der Ansprüche 1 bis 10, **dadurch gekennzeichnet, dass** sie auch eine Schneidführung für die Vorbereitung der Tibia zur Aufnahme der Schale (7) des Tibiaimplantats (1) nach Anspruch 1 umfasst.

13. Kit nach Anspruch 11, **dadurch gekennzeichnet, dass** es auch eine Schneidführung für die Vorbereitung der Tibia für die Aufnahme der Schale (7) des Tibiaimplantats (1) nach Anspruch 1 umfasst.

## Claims

1. An ankle prosthesis including a tibial implant (1), a talar implant (2) and an insert (3) with which said implants (1, 2) are articulated, according to which:
- the top face (6) of the tibial implant (1) comprises at least one anchoring fin (12) of the tibial implant (1) in the tibia of the patient, and the bottom face (15) of the tibial implant (1) is a surface articulated with the top surface (4) of the insert (3);
- the bottom face (18) of the talar implant (2) includes means for anchoring to the talar bone of the patient, and the top face (16) of the talar implant (2) is a surface articulated with the bottom surface (5) of the insert (3), said articulation surface being saddle-shaped and including a longitudinal central groove (17) in which the insert (3) can move longitudinally and rotate transversely;
**characterized in that** the top face (6) of the tibial implant (1) includes, on its anterior edge, a shield (7) intended to cover the bottom anterior portion of the tibia of the patient, and the tibial face (8) of which is inclined and forms an angle (α) with the horizontal of between 15° and 45°, and **in that** passages (10, 11) are provided in this shield (7) for bone screws that pass through the shield (7) in an ascending manner and open on the inclined tibial face (8) thereof.

2. The ankle prosthesis according to claim 1, **characterized in that** the bottom face (15) of the tibial implant (1) can be convex and in a sphere portion and have a curve radius (R), and **in that** the top face (4) of the insert (3) is then concave in a sphere portion and has a curve radius equal to that (R) of the bottom face (15) of the tibial implant (1).

3. The ankle prosthesis according to claim 1, **characterized in that** the bottom face (15) of the tibial implant (1) is convex and has a first curve radius (R1) in the anterior-posterior plane of the prosthesis, and a second curve radius (R2) in the medial-lateral plane of the prosthesis, and **in that** the top surface (4) of the insert (3) is concave and has the same curve radii (R1, R2) as the bottom face (15) of the tibial implant (1), the difference between said first (R1) and second (R2) curve radii being between 1 and 5% of said second curve radii (R2).

4. The ankle prosthesis according to one of claims 1 to 3, **characterized in that** the anchoring means of the talar implant can include at least one transverse fin (19) inclined relative to the bottom surface (18) of the talar implant (2) by an angle (β) oriented toward the rear of the prosthesis and the value of which is between 20 and 70°, preferably between 30 and 50°.

5. The ankle prosthesis according to claim 4, **characterized in that** its talar implant (2) includes at least two transverse fins (19) positioned side by side.

6. The ankle prosthesis according to claim 4 or 5, **characterized in that** the width of the transverse fin (19) or the sum of the widths of the transverse fins (19) placed side by side represents between 40 and 80% of the width of the talar implant (2).

7. The ankle prosthesis according to one of claims 1 to 6, **characterized in that** the at least one fin (12) for anchoring the tibial implant (1) in the tibia of the patient is in the general shape of a right-angled triangle, the hypotenuse (13) of which rests on the top face (6) of the tibial implant (1) and the small side (14) of which is oriented toward the front of the tibial implant (1).

8. The ankle prosthesis according to claim 7, **characterized in that** the length of the hypotenuse (13) of the tibial fin (12) represents between 50% and 80% of the total length of the top face (6) of the tibial implant (1).

9. The ankle prosthesis according to claim 7 or 8, **characterized in that** the upper corner of the tibial fin (12) is between 80° and 120°.

10. The ankle prosthesis according to one of claims 1 to 9, **characterized in that** the at least one anchoring fin (12) of the tibial implant (1) and the anchoring means of the talar implant (2) have not undergone any surface treatment intended to promote bone growth, whereas other portions of said implants (1, 2) intended to be in contact with the tibia or the talar bone have undergone such a surface treatment.

11. A kit comprising a plurality of ankle prostheses according to one of claims 1 to 10, **characterized in that** said prostheses have different sizes, but all have identical curve radii (R; R1, R2) on the bottom faces (15) of their tibial implants (1) and on the top faces (4) of their inserts (3).

12. A kit including at least one ankle prosthesis according to one of claims 1 to 10, **characterized in that** it also includes a cutting guide for preparing the tibia to receive the shield (7) of the tibial implant (1) according to claim 1.

13. The kit according to claim 11, **characterized in that** it also includes a cutting guide for preparing the tibia to receive the shield (7) of the tibial implant (1) according to claim 1.
